# EUROPEAN PATENT SPECIFICATION

(11) **EP 2 787 955 B1**
(45) Date of publication and mention of the grant of the patent: **07.02.2018**
(21) Application number: 12790544.6
(22) Date of filing: 21.11.2012
(51) Int. Cl.: A61K 8/34, A61K 8/36, A61K 8/368, A61Q 19/10, A61Q 17/00, A61K 8/46, A01N 31/04, A01N 31/08, B08B 1/00

(54) **AN ANTIMICROBIAL COMPOSITION**
ANTIMIKROBIELLE ZUSAMMENSETZUNG
COMPOSITION ANTIMICROBIENNE

(30) Priority: 09.12.2011 IN 3476MU2011; 26.01.2012 EP 12152564
(43) Date of publication of application: 15.10.2014
(73) Proprietor: Unilever N.V., 3013 AL Rotterdam (NL); Unilever PLC, London, Greater London EC4Y 0DY (GB)
(72) Inventor: JAYARAMAN, Sujatha, Whitefield Bangalore 560 066 (IN); APPAVOO, Shanti, Whitefield Bangalore 560 066 (IN); HIBARE, Sujitkumar Suresh, Whitefield Bangalore 560 066 (IN); IYER, Vidula, Whitefield Bangalore 560 066 (IN); SAJI, Maya Treesa, Whitefield Bangalore 560 066 (IN)
(74) Representative: van den Brom, Coenraad Richard
(86) International application number: PCT/EP2012/073193
(87) International publication number: WO 2013/083404

(56) References cited:
- WO-A1-2006/053458
- WO-A1-2010/046238
- WO-A1-2011/036048

## Description

### Technical Field

The present invention relates to a non-therapeutic method of disinfecting a surface and to an antimicrobial composition. It particularly relates to an antimicrobial composition comprising soap for disinfecting external surface of the human or animal bodies like skin.

### Background and Prior Art

Sanitizing and disinfecting compositions comprising chlorine and nascent oxygen based bleaching agents are known. Such compositions require rather long contact time to provide efficacious antimicrobial action. In practice, users, in particular children, do not spend long time in cleaning and as a result, cleaning with such compositions does not provide adequate prevention from surface or topical infection or adequate protection against diseases. The user, in spite of cleaning hands and other topical surfaces of the body with these compositions, is likely to have skin with relatively inadequate bacterial removal and this may cause contamination of further animate and/or inanimate surfaces and lead to spreading of pathogens and consequent diseases. Further, many antimicrobial actives in addition to abrasives are used in other applications like hard surface cleaning or cleaning of the oral cavity, but these actives generally require several minutes if not hours before effective antimicrobial action is effected.

WO2006/053458 (Givaudan) discloses bactericidal formulations comprising perfume ingredients active against gram-negative bacteria where the compositions contact the target site for short time, usually not longer than 30 seconds.

The present applicants in pursuit of solving this problem have disclosed in WO2010046238 a combination of essential oil actives thymol and terpineol that interact synergistically to provide anti-microbial activity in very fast times, in many cases as low as 15 seconds or lesser.

Using these actives in high amounts has the disadvantage that they are relatively expensive ingredients; additionally using high amounts of these have certain sensorial negatives like a strong odour that is not always appreciated by the consumer.

Accordingly it remains to be desired to prepare compositions having a high anti-microbial effect, even with low dosage of essential oil actives.

The present applicants have been working on further improving this technology and in an effort to find solutions to the problem of providing high antimicrobial efficacy at even further lower concentrations of essential oil actives, have arrived at the present invention. They have found that a combination of essential oil actives thymol and terpineol when used in the presence of specific hydrotropes are able to achieve the high antimicrobial efficacy at low concentration of the actives.

It is therefore an object of the invention to provide an antimicrobial composition, having good anti-microbial properties, at very low levels of essential oil actives.

### SUMMARY OF THE INVENTION

According to the first aspect of the invention there is provided an antimicrobial composition comprising
(a) an essential oil active mixture of 0.02 wt-% to less than 0.2 wt-% thymol and 0.05 wt-% to less than 0.5 wt-% terpineol, wherein wt% is expressed by weight of the composition; and
(b) 0.1 wt-% to 5 wt-% of a hydrotrope selected from the group consisting of sodium benzoate, sodium toluene sulphonate, sodium cumene sulphonate, sodium xylene sulphonate, sodium salicylate, or sodium acetate, and mixtures thereof.

According to a preferred aspect of the invention there is provided a composition according to the first aspect of the invention in the form of a soap bar composition comprising
(a) 40 to 80% total fatty matter; and
(b) 10 to 20% water.

Another aspect of the present invention relates to a non-therapeutic method for providing an anti-microbial effect to skin comprising the steps of:
(a) applying a composition of the first aspect, and
(b) waiting for at least 15 seconds.

According to yet another aspect of the invention there is provided a non-therapeutic use of a composition according to the invention for fast reduction in microbial count.

### DETAILED DESCRIPTION OF THE INVENTION

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated.

Except in the operating and comparative examples, or where otherwise explicitly indicated, all numbers in this description indicating amounts of material or conditions of reaction, physical properties of materials and/or use are to be understood as modified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated.

By an antimicrobial composition as used herein, is meant to include a composition for cleaning and disinfecting topical areas e.g. skin and/or hair of mammals, especially humans. Such a composition may be generally classified as leave-on or rinse off, and includes any product applied to a human body for also improving appearance, cleansing, odor control or general aesthetics. It is more preferably a rinse off product.

The composition of the present invention may be in the form of a liquid but may be modified to include a lotion, cream, foam or gel, or toner, or applied with an implement or via a face mask, pad or patch. Preferably the composition is in the solid form. "Skin" as used herein is meant to include skin on the face and body (e.g., neck, chest, back, arms, underarms, hands, legs, buttocks and scalp). The composition of the invention is also of relevance to applications on any other keratinous substrates of the human body other than skin e.g. hair where products may be formulated with specific aim of providing disinfection and cleaning.

The composition of the invention may be formulated in any one of the product form listed hereafter, each of which are defined below. By a soap bar is meant a composition that comprises a soap i.e. a salt of fatty acid in a shaped solid form. Soaps may be prepared by the milled and plodded route or by the melt cast route. By a liquid personal cleaning composition is meant a composition in liquid form that is used for various personal cleaning applications like hand washing, body washing, face washing or cleaning of hair. By an anti-septic liquid is meant a composition, usually in transparent form which may be coloured or may be substantially colourless that is used to disinfect various animate and inanimate surfaces. The anti-septic liquid is usually used after dilution with water usually in the weight ratio of 1:1 to 1:200 preferably in a ratio of 1:10 to 1:80. By a hard surface cleaning composition which may be in the bar, liquid or gel form, is meant a composition to clean hard surfaces in homes and other public places e.g. floors, kitchen tops, furniture, utensils and crockery. The composition of the present invention may be used to clean and disinfect various surfaces by way of the above mentioned forms.

The composition of the invention comprises a mixture of essential oil actives thymol and terpineol, and a hydrotrope. This composition is especially useful since the concentration of essential oil antimicrobial actives that needs to be used is low. The composition comprises the essential oil actives thymol and terpineol. Additional essential oil actives viz. eugenol, geraniol or a mixture thereof, may also be included. Even further more preferred is a mixture of thymol, terpineol and eugenol.

### Thymol

The structure of thymol is given below:

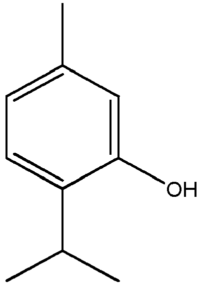

The composition of the invention comprises thymol at 0.02 to less than 0.2% by weight of the composition. Thymol may be added to the composition in purified form. Alternatively, thyme oil or thyme extract comprising thymol may be added to the composition, while ensuring that thymol is present in the desired concentration in the composition of the present invention. Thyme oil or thyme extract is obtained from the thyme plant. Thyme plant refers to a plant belonging be genus *Thymus* and includes but is not limited to the following species: *Thymus vulgaris, Thymus zygis, Thymus satureoides, Thymus mastichina, Thymus broussonetti, Thymus maroccanus, Thymus pallidus, Thymus algeriensis, Thymus serpyllum, Thymus pulegoide,* and *Thymus citriodorus.*

### Terpineol

The structure of a terpineol compound is given below:

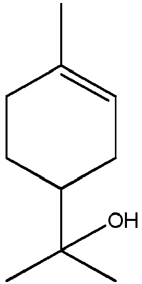

The terpineol is preferably selected from alpha-terpineol, beta-terpineol, gammaterpineol or mixtures thereof. It is particularly preferred that the terpineol is alpha-terpineol. Terpineol may be added to the antimicrobial composition in purified form. Alternatively pine oil comprising terpineol may be added to the antimicrobial composition while ensuring that terpineol is present in the desired concentration in the composition of the present invention. The composition comprises terpineol at 0.05 to less than 0.5% by weight of the composition.

The composition preferably also comprises 0.03 to 0.4% eugenol, by weight of the composition.

The composition comprises a hydrotrope which is selected from the group consisting of sodium benzoate, sodium toluene sulphonate, sodium cumene sulphonate, sodium xylene sulphonate, sodium salicylate, sodium acetate, and mixtures thereof. The more preferred hydrotropes are sodium benzoate, sodium acetate and sodium salicylate. The hydrotrope is present in 0.1 to 5%, by weight of the composition.

The antimicrobial composition of the invention may be used to develop various personal care and household care products. Examples include personal care compositions in the form of creams, lotions and gels which may provide various other benefits like moisturization, sun screening and skin lightening benefits, malodour control and antiperspirancy. The antimicrobial benefits are afforded by the composition of the invention in very fast times e.g. in less than 5 minutes, often in less than one minutes, in some cases in less than 30 seconds and in certain other cases in less than 15 seconds. This fast acting antimicrobial composition is especially suited for incorporation in wash-off products e.g. soaps in the form of bars, liquids and gels. These products may be used for personal cleansing e.g. as personal wash soap bars, body wash liquids, shower gels, hand wash liquids, gels and lotions, and as face wash products. It is preferred that the composition of the invention is formulated to have a pH of 3 to 11 preferably 5 to 11 where the efficacy of the synergistic interaction between the antimicrobial actives, the polymer and the hydrotrope is seen to be maxiumum. The composition may also be used for providing disinfection benefits to other parts of the body like hair and the oral cavity. Thus the composition may be used to formulate shampoos, conditioners and mouthwashes.

The composition of the invention may be used for cleaning hard surfaces and thus may be formulated as a floor cleaner, toilet cleaner or a gel or emulsion for cleaning surfaces in the kitchen like table tops and to clean ovens.

Particularly preferred carriers for formulating the composition of the invention in the various products mentioned above are water or oil/solvent, more preferred carrier being a mixture of water and oil. In most of the envisaged applications like personal care/washing, oral care and hard surface cleaning, the antimicrobial composition may be formulated in an aqueous base (water being carrier) e.g. products in gel format or in purely oil/solvent base e.g. products in anhydrous stick form or propellant containing products. However, most preferred product format has an emulsion base (water and oil being the carrier) e.g. soap products in liquid, solid, lotion or semisolid form for hand wash, face wash, body wash, or shaving applications; toothpaste/ dentifrices for oral care applications or products for hard surface cleaning in bars or liquids form.

The antimicrobial composition preferably comprises 1 to 80% surfactant. In general, the surfactants may be chosen from the surfactants described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used.

A particularly preferred surfactant is soap. Soap is a suitable surfactant for personal washing applications of the antimicrobial composition of the invention. The soap is preferably C8-C24 soap, more preferably C10-C20 soap and most preferably C12-C16 soap. The soap may or may not have one or more carbon-carbon double bond or triple bond. The cation of the soap can be alkali metal, alkaline earth metal or ammonium. Preferably, the cation of the soap is selected from sodium, potassium or ammonium. More preferably the cation of the soap is sodium or potassium.

The soap may be obtained by saponifying a fat and/or a fatty acid. The fats or oils generally used in soap manufacture may be such as tallow, tallow stearines, palm oil, palm stearines, soya bean oil, fish oil, castor oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soyabean, castor etc. The fatty acid soaps can also be synthetically prepared (e.g. by the oxidation of petroleum or by the hydrogenation of carbon monoxide by the Fischer-Tropsch process). Resin acids, such as those present in tall oil, may be used. Naphthenic acids are also suitable.

Tallow fatty acids can be derived from various animal sources and generally comprise about 1-8% myristic acid, about 21-32% palmitic acid, about 14-31% stearic acid, about 0-4% palmitoleic acid, about 36-50% oleic acid and about 0-5% linoleic acid. A typical distribution is 2.5% myristic acid, 29% palmitic acid, 23% stearic acid, 2% palmitoleic acid, 41.5% oleic acid, and 3% linoleic acid. Other similar mixtures, such as those from palm oil and those derived from various animal tallow and lard are also included.

Coconut oil refers to fatty acid mixtures having an approximate carbon chain length distribution of 8% C₈, 7% C₁₀, 48% C₁₂, 17% C₁₄, 8% C₁₆, 2% C₁₈, 7% oleic and 2% linoleic acids (the first six fatty acids listed being saturated). Other sources having similar carbon chain length distributions, such as palm kernel oil and babassu kernel oil, are included within the term coconut oil.

A typical fatty acid blend consisted of 5 to 30% coconut fatty acids and 70 to 95% fatty acids ex hardened rice bran oil. Fatty acids derived from other suitable oils/fats such as groundnut, soybean, tallow, palm, palm kernel, etc. may also be used in other desired proportions. The soap, when present in solid forms of the present invention, is present in an amount of 30 to 90%, preferably from 50 to 85%, more preferably 55 to 75% by weight of the composition. The soap, when present in liquid forms of the composition is present in 0.5 to 20%, preferably from 1 to 10% by weight of the composition.

The composition of the invention may comprise soap which may be in the form of a solid, soft solid, gel, emulsion, or liquid. When in solid form, the composition is preferably a bar. The soap bar may be prepared by the milled and plodded route or may be prepared using the melt cast route. Of the two routes the milled and plodded route is more preferred for preparing a soap bar of the present invention.

Personal wash compositions are available in various forms such as soap bars, transparent soap bars including cast-bars, liquid soaps including liquid hand wash compositions, creams and gel based products. Commercial soap compositions have one or more "soaps", which has the meaning as normally understood in the art; salts of mono carboxylic fatty acids. The counterions of the salts are generally sodium, potassium, ammonium or alkanolammonium ions, but other suitable ions known in the art may also be used. Compositions based on soaps, i.e. soap bars generally contain anywhere from 15 to 80% by weight alkali metal salt of fatty acids, depending on whether the soap is in solid or liquid form, which accounts for the total fatty matter (TFM), the remainder being water (about 10-20%) and other ingredients such as metal ion chelators, color, perfume, preservatives etc. Structurants and fillers are also frequently added to such compositions in small amount to replace some of the soap, while retaining the desired properties of the product. Soaps having TFM content of about 70 are called "toilet soaps", whereas those having TFM of about 40 are called "bathing bars".

Thus, according to a preferred aspect of the present invention, the composition according to the invention is in the form of a soap bar composition comprising 40 to 80% total fatty matter; and 10 to 20% water. The soap bar may comprise an additional essential oil active eugenol.

The composition may further comprise various additional ingredients known to a person skilled in the art. Such additional ingredients include but are not limited to: perfumes, pigments, preservative, emollients, sunscreens, emulsifiers, gelling agents, or thickening agents.

Water is a preferred carrier, in most products prepared with the composition of the invention. When water is present, it is preferably present in at least 1%, more preferably at least 2%, further more preferably at least 5% by weight of the composition. Water is preferably present in 10 to 80% in most formulation prepared with the composition of the invention.

According to another aspect, inorganic particulate material is also a suitable carrier. When inorganic particulate material is the carrier, the antimicrobial composition is in a solid form. Preferably the inorganic particulate material is talc. When the inorganic particulate material is talc, the solid antimicrobial composition is particularly useful as a talcum powder for application on face or body.

According to one preferred aspect, the invention provides for non-therapeutic benefits.

Thus, according to yet another aspect of the invention there is provided a non-therapeutic use according to claim 9. The standard procedure BS EN 1040 has been used to measure microbial count with minor modification on the desired contact time of the microorganisms with the antimicrobial active.

Another aspect of the present invention provides for a non-therapeutic method for providing an anti-microbial effect to skin comprising the steps of (a) applying a composition of the first aspect to skin and (b) waiting for at least 15 seconds.

The non-therapeutic method preferably comprises the step of wiping or rinsing the composition from the substrate after the waiting step.

The invention will now be illustrated with the help of the following non-limiting examples.

### EXAMPLES

### Examples 1-8: Compositions to demonstrate the synergistic interaction of the ingredients of the invention

Compositions as shown in Table - 1 were prepared and the compositions were tested for antimicrobial efficacy in a 15 seconds contact test, using the following protocol:

### Protocol: Contact kill Assay (15 second contact kill)

The test bacteria *E.coli* ATCC 10536 was grown overnight in TSB broth (Difco - 30gpl) at 37°C for 16hrs. 2ml of this was sub-cultured in 40ml of fresh TSB broth and kept for growing for 4hrs at 37°C. Then the culture was processed by spinning at 4000rpm for 5min, washed twice and then collecting the cells. The cell density was adjusted at 620 nm to get the final count of 10⁸ cfu/ml (0.8 OD). The test solutions/formulations were prepared and kept for 3 hours for maturation.

9 ml of the test solution was taken in a sample container and 1 ml of processed culture was added to it. After 15 second contact time, 1 ml of the above mixture was immediately neutralized in D/E broth (Difco - 39gpl). Serial dilution was done in D/E broth and plated on TSA (Difco - 40gpl) in duplicates. In case of the control, 1 ml of test culture was added to 9 ml of saline and was serially diluted and plated on TSA. After solidification, the plates were incubated at 37°C for 48 hrs. The residual colonies after 48 hours of incubation were counted and the efficacy was calculated by comparing with control.

**Table - 1**

| Examples | Thymol wt% | Terpineol wt% | Hydrotrope | | Water | Log reduction |
|---|---|---|---|---|---|---|
| | | | type | wt% | | |
| Ex-1 | 0.2 | 0.5 | - | - | To 100 | 7.2 |
| Ex-2 | 0.025 | 0.0625 | - | - | To 100 | 0.3 |
| Ex-3 | - | - | Sodium benzoate | 2 | To 100 | 0.2 |
| Ex-4 | 0.025 | 0.0625 | Sodium benzoate | 2 | To 100 | 7.2 |
| Ex-5 | 0.025 | 0.0625 | Sodium acetate | 2 | To 100 | 7.2 |
| Ex-6 | 0.025 | 0.0625 | Sodium salicylate | 2 | To 100 | 6.1 |
| Ex-7 | 0.025 | 0.0625 | STS^{*} | 2 | To 100 | 5.5 |
| Ex-8 | 0.025 | 0.0625 | SCS^{#} | 2 | To 100 | 4.6 |

| | | | | | | |
|---|---|---|---|---|---|---|
| *STS is sodium para toluene sulphonate ^{#}SCS is sodium para cumene sulphonate | | | | | | |

The data in Table - 1 indicates that when thymol and terpineol are used at high concentration (Example -1 above and as claimed in WO2010046238) the antimicrobial efficacy is high. However when low concentration of these actives are used, one does not get the desired high anti-microbial efficacy. With the use of the compositions as per the invention (Examples 4 to 8), the ingredients interact synergistically to provide the high desired anti-microbial efficacy.

### Examples 9 to 14: Evaluation of the in-vivo efficacy of soap bar compositions of the invention

Compositions as shown in Table - 2 and 3 were prepared. The compositions were used on subjects' hands for washing and the washings were collected and analysed for bacterial counts.

### Soap Application:

Subjects are given a controlled wash of both left and right forearms. The required soap is applied on either forearm of the panelists. The test area of left/right forearm is first moistened / wetted with tap water. The soap is applied to the test area with a gloved hand for 15 seconds. The area is lathered again for 15 seconds. The subject then rinsed the test area with tap water for 30 seconds to remove all the lather.

### Post-wash sampling:

Samples are collected from both left and right forearm sites, immediately after soap application.

The area to be sampled is delineated by a sterile sampling cylinder. The cylinder is pressed firmly against the skin surface during sampling to ensure that the washing fluid did not leak from the sampling site. A 1.5-ml portion of sampling fluid is added to the cylinder and the entire area is scrubbed with moderate pressure using a sterile glass rod for one minute. The sampling fluid is removed and pipetted into a sterile sample tube. This procedure is repeated with a fresh aliquot of sampling fluid, and the samples so collected are pooled.

### Enumeration of samples for bacterial counts:

The cup scrub samples collected are diluted serially and three appropriate dilutions are plated in duplicate using the pour plate method on CY agar plates, a total of 6 plates are used per sample. The plates are incubated at 37°C for 48 hours. The colonies are counted and recorded in the data sheets. Bacterial count/ml is calculated considering, dilution factor as per standard microbiological counting procedures.

The antimicrobial efficacy of a sample was compared to the efficacy of a control soap bar which was prepared without the essential ingredients of the invention. The log difference in microbial count is shown in Table -2 and 3. The data is an average of 11 replicates.

**Table - 2**

| Ingredients | Ex - 9, wt% | Ex - 10, wt% | Ex - 11, wt% |
|---|---|---|---|
| Thymol | 0.1 | - | 0.1 |
| Terpineol | 0.1 | - | 0.1 |
| Sodium benzoate | - | 4.0 | 4.0 |
| 68% TFM Soap | 75 | 75 | 75 |
| Water | 14 | 14 | 14 |
| Other minors like Talc, glycerine and perfume | To 100 | To 100 | To 100 |
| Difference in log reduction | 0.24 | 0.17 | 0.69 |

**Table - 3**

| Ingredients | Ex - 12 wt% | Ex - 13 wt% | Ex - 14 wt% |
|---|---|---|---|
| Thymol | 0.1 | - | 0.1 |
| Terpineol | 0.1 | - | 0.1 |
| Sodium salicylate | - | 4.0 | 4.0 |
| 68% TFM Soap | 75 | 75 | 75 |
| Water | 14 | 14 | 14 |
| Other minors like Talc, glycerine and perfume | To 100 | To 100 | To 100 |
| Difference in log reduction | 0.24 | 0.49 | 0.87 |

The data in Table - 2 and 3 indicate that a soap bar composition of the invention (Example 11 and 14) provides for vastly improved antimicrobial efficacy as compared to including only one of the two essential ingredients of the composition.

The invention thus provides for a composition that exhibits vastly improved antimicrobial efficacy by synergistic interaction of the ingredients and this is achieved using very low concentration of the essential oil actives.

### Examples 15-18: Compositions outside the invention

Compositions as shown in Table - 4 were prepared and they were tested for antimicrobial efficacy in a 15 seconds contact test, using the protocol as used for examples 1 to 8. The data on the log reduction provided by the various samples is shown in Table -4.

**Table - 4**

| Examples | Thymol wt% | Terpineol wt% | Sodium xylene sulphonate, wt% | Log reduction |
|---|---|---|---|---|
| Ex-15 | 0.003 | - | 12 | 0 |
| Ex-16 | | 0.003 | 12 | 0 |
| Ex-17 | 0.02 | - | 3 | 0.1 |
| Ex-18 | | 0.02 | 3 | 0 |

The data in Table -4 indicate that using a single essential active as disclosed in WO2006/053458 (Example -15), one does not get the fast acting antibacterial activity. Further obvious extensions of the teachings of the above publication (Examples 16 to 18) are also equally ineffective.

## Claims

1. An antimicrobial composition comprising
(a) an essential oil active mixture of 0.02 wt-% to less than 0.2 wt-% thymol and 0.05 wt-% to less than 0.5 wt-% terpineol, wherein wt% is expressed by weight of the composition; and
(b) 0.1 wt-% to 5 wt-% of a hydrotrope selected from the group consisting of sodium benzoate, sodium toluene sulphonate, sodium cumene sulphonate, sodium xylene sulphonate, sodium salicylate, sodium acetate, and mixtures thereof.

2. A composition as claimed in claim 1 wherein said hydrotrope is selected from the group consisting of sodium benzoate, sodium salicylate, sodium acetate, and mixtures thereof.

3. A composition as claimed in any one of the previous claims in the form of a soap bar composition comprising
(a) 40 to 80% total fatty matter; and
(b) 10 to 20% water.

4. A soap bar composition as claimed in claim 3 comprising 0.1 to 5% of the hydrotrope.

5. A soap bar composition as claimed in claim 3 or 4 comprising an additional essential oil active eugenol.

6. A composition as claimed in any one of the preceding claims, wherein the pH of the composition is between 5 and 11.

7. A non-therapeutic method for providing an anti-microbial effect to skin comprising the steps of:
(a) applying a composition as claimed in any one of the preceding claims to skin, and
(b) waiting for at least 15 seconds.

8. A non-therapeutic method as claimed in claim 7, wherein the composition is wiped or rinsed from the substrate after step 'b'.

9. Non-therapeutic use of a composition according to any one of claims 1 to 3 for reduction in microbial count in less than 5 minutes.

## Patentansprüche

1. Antimikrobielle Zusammensetzung, umfassend
(a) eine ätherisches Öl-Aktivmischung von 0,02 Gew.-% bis weniger als 0,2 Gew.-% Thymol und 0,05 Gew.-% bis weniger als 0,5 Gew.-% Terpineol, wobei Gew.-% in Gewicht der Zusammensetzung ausgedrückt ist, und
(b) 0,1 Gew.-% bis 5 Gew.-% eines Hydrotrops, ausgewählt aus der aus Natriumbenzoat, Natriumtoluolsulfonat, Natriumcumolsulfonat, Natriumxylolsulfonat, Natriumsalicylat, Natriumacetat und Mischungen davon bestehenden Gruppe.

2. Zusammensetzung, wie im Anspruch 1 beansprucht, wobei das Hydrotrop aus der aus Natriumbenzoat, Natriumsalicylat, Natriumacetat und Mischungen davon bestehenden Gruppe ausgewählt wird.

3. Zusammensetzung, wie in irgendeinem der vorhergehenden Ansprüche beansprucht, in Form einer Seifenstückzusammensetzung, umfassend
(a) 40 bis 80% gesamte Fettsubstanz und
(b) 10 bis 20% Wasser.

4. Seifenstückzusammensetzung, wie im Anspruch 3 beansprucht, umfassend 0,1 bis 5% des Hydrotrops.

5. Seifenstückzusammensetzung, wie im Anspruch 3 oder 4 beansprucht, umfassend ein zusätzliches ätherisches Öl-Aktiveugenol.

6. Zusammensetzung, wie in irgendeinem der vorhergehenden Ansprüche beansprucht, wobei der pH der Zusammensetzung zwischen 5 und 11 liegt.

7. Nicht-therapeutisches Verfahren zur Schaffung eines antimikrobiellen Effekts auf Haut, umfassend die Schritte:
(a) Auftragen einer Zusammensetzung, wie in irgendeinem der vorhergehenden Ansprüche beansprucht, auf Haut und
(b) Warten für mindestens 15 Sekunden.

8. Nicht-therapeutisches Verfahren, wie im Anspruch 7 beansprucht, wobei die Zusammensetzung von dem Substrat nach Schritt 'b' abgewischt oder abgespült wird.

9. Nicht-therapeutische Verwendung einer Zusammensetzung nach irgendeinem der Ansprüche 1 bis 3 zum Senken der Keimzahl in weniger als 5 Minuten.

## Revendications

1. Composition antimicrobienne comprenant
(a) un mélange de principes actifs d'huiles essentielles de 0,02 % en masse à moins de 0,2 % en masse de thymol et de 0,05 % en masse à moins de 0,5 % en masse de terpinéol, où % en masse est exprimé en masse de la composition ; et
(b) 0,1 % en masse à 5 % en masse d'un hydrotrope choisi dans le groupe constitué de benzoate de sodium, toluènesulfonate de sodium, cumènesulfonate de sodium, xylènesulfonate de sodium, salicylate de sodium, acétate de sodium, et mélanges de ceux-ci.

2. Composition selon la revendication 1, dans laquelle ledit hydrotrope est choisi dans le groupe constitué de benzoate de sodium, salicylate de sodium, acétate de sodium, et mélanges de ceux-ci.

3. Composition selon l'une quelconque des revendications précédentes, dans la forme d'une composition de barre de savon comprenant
(a) de 40 à 80 % de matière grasse totale ; et
(b) de 10 à 20 % d'eau.

4. Composition de barre de savon selon la revendication 3 comprenant de 0,1 à 5 % de l'hydrotrope.

5. Composition de barre de savon selon la revendication 3 ou 4 comprenant de l'eugénol de principe actif d'huile essentielle supplémentaire.

6. Composition selon l'une quelconque des revendications précédentes, où le pH de la composition est de 5 à 11.

7. Procédé non-thérapeutique pour fournir un effet antimicrobien à la peau comprenant les étapes de :
(a) application d'une composition selon l'une quelconque des revendications précédentes à la peau, et
(b) attente pendant au moins 15 secondes.

8. Procédé non-thérapeutique selon la revendication 7, dans lequel la composition est essuyée ou rincée du substrat après l'étape 'b'.

9. Utilisation non-thérapeutique d'une composition selon l'une quelconque des revendications 1 à 3 pour une réduction du compte de microbes en moins de 5 minutes.
